# EUROPÄISCHE PATENTANMELDUNG

(11) **EP 0 542 132 A1**
(43) Veröffentlichungstag der Anmeldung: **19.05.1993**
(21) Anmeldenummer: 92118947.8
(22) Anmeldetag: 05.11.1992
(51) Int. Cl.: C07C 68/02, C07C 69/96

(54) **Verfahren zur Herstellung von Chlorameisensäurearylestern**

(30) Priorität: 15.11.1991 DE 4137557
(71) Anmelder: BASF Aktiengesellschaft, D-67063 Ludwigshafen (DE)
(72) Erfinder: Koehler, Hermann, Dr., W-6719 Bobenheim (DE); Wettling, Thomas, Dr., W-6703 Limburgerhof (DE); Franzischka, Wolfgang, Dr., W-6710 Frankenthal (DE); Hupfer, Leopold, Dr., W-6701 Friedelsheim (DE)

(57) **Zusammenfassung**

Verfahren zur Herstellung von Chlorameisensäurearylestern der allgemeinen Formel I 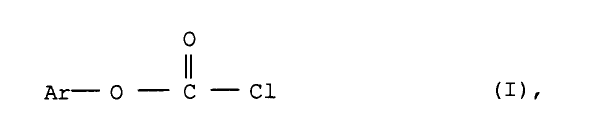
in der
- Ar: ein gegebenenfalls ein- bis fünffach durch C₁- bis C₂₀-Alkyl, C₂- bis C₂₀-Alkenyl, C₃- bis C₂₀-Cycloalkyl, C₄- bis C₂₀-Alkyl-cycloalkyl, C₁- bis C₂₀-Halogenalkyl, C₁- bis C₂₀-Alkoxy, C₁- bis C₂₀-Alkylthio, Halogen, Cyano, C₂- bis C₂₀-Alkylcarbonyloxy, C₃- bis C₂₀-Carboalkoxy, Formyl, C₂- bis C₂₀-Dialkylamino, Aryl, Aryloxy, Arylthio, Aroyl, C₇- bis C₂₀-Aralkyl, C₇- bis C₂₀-Aralkoxy, Arylsulfonyl und/oder C₇- bis C₂₀-Aralkylthio und/oder ein- bis zweifach durch Chlorformyl und/oder Nitro substituiertes Aryl
bedeutet, aus Phosgen und Phenolen der allgemeinen Formel II

Ar-OH (II),

in der Ar die obengenannten Bedeutungen hat sowie gegebenenfalls ein- bis zweifach Hydroxygruppen trägt, indem man die Umsetzung bei einer Temperatur von 40 bis 250°C und Drücken von 0,01 bis 50 bar in Gegenwart von Pyridinverbindungen der allgemeinen Formel III 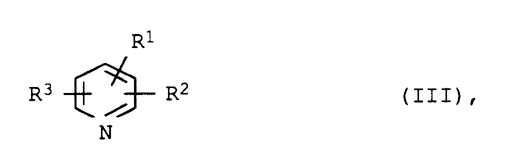
in der
- R¹: C₅- bis C₂₀-Alkyl und
- R², R³: Wasserstoff oder C₁- bis C₂₀-Alkyl
bedeuten, durchgeführt.

## Beschreibung

Die Erfindung betrifft ein Verfahren zur Herstellung von Chlorameisensäurearylestern durch Umsetzung von Phenolen mit Phosgen in Gegenwart von Pyridinverbindungen.

Es ist bereits bekannt, Alkohole oder Phenole mit Phosgen zu den entsprechenden Chlorameisensäureestern umzusetzen (Ullmanns Enzyklopädie der technischen Chemie, 4. Auflage, Band 9, Seite 381, Verlag Chemie 1975). Während aliphatische Alkohole auch ohne Zusätze mit Phosgen reagieren können, müssen bei der Umsetzung von Phosgen mit Phenolen Zusätze angewendet werden, beispielsweise solche, die in der Lage sind, die freiwerdende Salzsäure zu binden.

Der Zusatz von organischen Stickstoffbasen ist aus der DE-A-30 00 524, und der US-A-3,211,776 (organische Ammoniumsalze) bekannt. Die Aufarbeitung wird in diesen Fällen dadurch erschwert, daß die Ammoniumsalze häufig auskristallisieren.

Der vorliegenden Erfindung lag daher die Aufgabe zugrunde, den zuvor genannten Nachteilen abzuhelfen.

Demgemäß wurde ein neues und verbessertes Verfahren zur Herstellung von Chlorameisensäurearylestern der allgemeinen Formel I 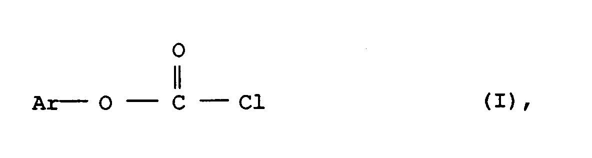
in der
- Ar: ein gegebenenfalls ein- bis fünffach durch C₁- bis C₂₀-Alkyl, C₂- bis C₂₀-Alkenyl, C₃- bis C₂₀-Cycloalkyl, C₄- bis C₂₀-Alkyl-cycloalkyl, C₁- bis C₂₀-Halogenalkyl, C₁- bis C₂₀-Alkoxy, C₁- bis C₂₀-Alkylthio, Halogen, Cyano, C₂- bis C₂₀-Alkylcarbonyloxy, C₃- bis C₂₀-Carboalkoxy, Formyl, C₂- bis C₂₀-Dialkylamino, Aryl, Aryloxy, Arylthio, Aroyl, C₇- bis C₂₀-Aralkyl, C₇- bis C₂₀-Aralkoxy, Arylsulfonyl und/oder C₇- bis C₂₀-Aralkylthio und/oder ein- bis zweifach durch Chlorformyl und/oder Nitro substituiertes Aryl
bedeutet, aus Phosgen und Phenolen der allgemeinen Formel II

Ar-OH (II),

in der Ar die obengenannten Bedeutungen hat sowie gegebenenfalls ein- bis zweifach Hydroxygruppen trägt, gefunden, welches dadurch gekennzeichnet ist, daß man die Umsetzung bei einer Temperatur von 40 bis 250°C und Drücken von 0,01 bis 50 bar in Gegenwart von Pyridinverbindungen der allgemeinen Formel III 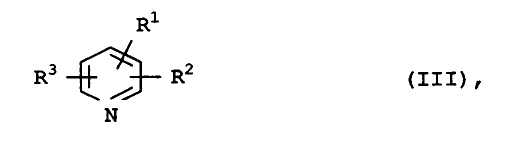
in der
- R¹: C₅- bis C₂₀-Alkyl und
- R²,R³: Wasserstoff oder C₁- bis C₂₀-Alkyl
bedeuten, durchgeführt.

Allgemein wird bei dem erfindungsgemäßen Verfahren ein Phenol der allgemeinen Formel II in Gegenwart einer Pyridinverbindung der Formel III mit Phosgen zu dem entsprechenden Chlorformiat umgesetzt.

Das für die Reaktion benötigte Phosgen kann gasförmig oder in flüssiger Form zugegeben werden.

Das erfindungsgemäße Verfahren kann kontinuierlich oder diskontinuierlich durchgeführt werden. Ein kontinuierliches Verfahren kann beispielsweise im Reaktionsrohr, in einer Rührkesselkaskade, in einem Schlaufenreaktor oder in einer Gegenstromkolonne durchgeführt werden.

Der Katalysator kann dabei je nach gewählter Verfahrensvariante entweder vorgelegt oder kontinuierlich zugegeben werden, wobei er z.B. auch im Phenol II gelöst sein kann.

Man kann das erfindungsgemäße Verfahren kann bei Temperaturen von 40 bis 250°C, bevorzugt von 60 bis 150°C, besonders bevorzugt von 80 bis 140°C und Drücken von 0,01 bis 50 bar, bevorzugt 0,5 bis 5 bar, besonders bevorzugt bei Normaldruck (Atmosphärendruck), vorzugsweise in homogener flüssiger Phase durchgeführen. Homogene flüssige Phasen sind beispielsweise die Schmelze der Phenole II oder eine Lösung der Phenole II in einem inerten Lösungsmittel.

Die Pyridinverbindung III kann in einer Menge von beispielsweise 0,01 bis 25 Mol-%, bevorzugt 0,1 bis 15 Mol-%, besonders bevorzugt 0,5 bis 10 Mol-%, bezogen auf das Phenol II eingesetzt werden.

Das Molverhältnis von Phosgen zum Phenol II beträgt 1:1 bis 2:1, bevorzugt 1:1 bis 1,5:1, besonders bevorzugt 1:1 bis 1,3:1. Die Verwendung von größeren Überschüssen als 2:1 ist zwar möglich, aber allgemein nicht angezeigt, da sie keine weiteren Vorteile bringt. Die Verwendung von weniger als der stöchiometrischen Menge Phosgen ist möglich, bringt aber im allgemeinen keinen Vorteil, da hierbei nicht umgesetztes Phenol abgetrennt werden muß und die Ausbeute sinkt.

Als Lösungsmittel eignen sich beispielsweise ein aliphatischer oder aromatischer Kohlenwasserstoff wie Pentan, Hexan, Cyclohexan, Toluol, Xylol oder Benzol, halogenierte Kohlenwasserstoffe, wie Trichlorethan, Chlorbenzol oder Dichlorbenzol oder Ester, wie Ethylacetat oder Butylacetat oder das durch Phosgenierung entstehende Chlorformiat des entsprechenden Phenols, bevorzugt sind aromatische Kohlenwasserstoffe wie Benzol, Toluol, die Xylole und 1,2-Dichlorbenzol.

Die Reaktion kann in einer weiteren bevorzugten Weise entweder ohne Lösungsmittel nur in der Schmelze des Phenols, oder unter Verwendung der z.B. durch Phosgenierung der beschriebenen Phenole entstehenden Chlorformiate als Lösungsmittel durchgeführt werden. Hierdurch wird eine höhere Raum-Zeit-Ausbeute erzielt als beim Arbeiten in Gegenwart anderer Lösungsmittel, da das Abdestillieren des Lösungsmittels entfällt. Jedoch kann das Arbeiten im Lösungsmittel dann vorteilhaft sein, wenn der Schmelzpunkt des einzusetzenden Phenols oder des entsprechenden Chlorformiats oberhalb der angestrebten Reaktionstemperatur liegt und das Phenol daher, zumindest zu Beginn der Reaktion, ohne Gegenwart des Lösungsmittels nur langsam mit dem Phosgen reagieren würde. Die Gegenwart eines Lösungsmittels kann auch zur Beherrschung und Abführung der Reaktionswärme der exothermen Reaktion günstig sein.

Die Aufarbeitung des Reaktionsgemisches kann z.B. durch Destillation erfolgen. Der Destillationsrückstand enthält die Pyridinverbindung III, die für eine weitere Umsetzung des erfindungsgemäßen Verfahrens eingesetzt werden kann.

Die Substituenten Ar, R¹, R² und R³ in den Formeln I, II und III haben folgende Bedeutungen:
Ar
- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- ein gegebenenfalls ein- bis fünffach durch einen der folgenden Reste substituiertes Aryl;
- C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C1- bis C4-Alkyl wie Methyl, Ethyl, n-Propyl, iso-Propyl, n-Butyl, iso-Butyl, sec.-Butyl und tert.-Butyl,
- C₂- bis C₂₀-Alkenyl, bevorzugt C₂- bis C₈-Alkenyl wie Vinyl, Allyl, But-2-en-1-yl, But-4-en-1-yl, But-4-en-2-yl, Pent-2-en-1-yl und 2,2-Dimethylpent-1-en-1-yl,
- C₃- bis C₂₀-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
- C₄- bis C₂₀-Alkyl-cycloalkyl, bevorzugt C₄- bis C₁₂-Cycloalkyl-alkyl wie Cyclopentyl-methyl und Cyclohexylmethyl,
- C₁- bis C₂₀-Halogenalkyl, bevorzugt C₁- bis C₄-Halogenalkyl, besonders bevorzugt C₁- bis C₄-Fluor-, Chlor- und/oder Bromalkyl wie Fluormethyl, Difluormethyl, Trifluormethyl, Chlormethyl, Dichlormethyl, Trichlormethyl, Brommethyl, Dibrommethyl und Tribrommethyl,
- C₁- bis C₂₀-Alkoxy, bevorzugt C₁- bis C₁₂-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, n-Nonoxy, iso-Nonoxy, n-Decoxy, iso-Decoxy, n-Undecoxy, iso-Undecoxy, n-Dodecoxy und iso-Dodecoxy, besonders bevorzugt C₁- bis C₈-Alkoxy wie Methoxy, Ethoxy, n-Propoxy, iso-Propoxy, n-Butoxy, iso-Butoxy, sec.-Butoxy, tert.-Butoxy, n-Pentoxy,iso-Pentoxy, sec.-Pentoxy, neo-Pentoxy, 1,2-Dimethylpropoxy, n-Hexoxy, iso-Hexoxy, sec.- Hexoxy, n-Heptoxy, iso-Heptoxy, n-Octoxy, iso-Octoxy,
- C₁- bis C₂₀-Alkylthio, bevorzugt C₁- bis C₈-Alkyl wie Methylthio, Ethylthio, n-Propylthio, iso-Propylthio, n-Butylthio, iso-Butylthio, sec.-Butylthio, tert.-Butylthio, n-Pentylthio, iso-Pentylthio, sec.-Pentylthio, neo-Pentylthio, 1,2-Dimethylpropylthio, n-Hexylthio, iso-Hexylthio, sec.-Hexylthio, n-Heptylthio, iso-Heptylthio, n-Octylthio, iso-Octylthio, besonders bevorzugt C₁- bis C₄-Alkyl wie Methylthio, Ethylthio, n-Propylthio, iso-Propylthio, n-Butylthio, iso-Butylthio, sec.-Butyl und tert.-Butylthio,
- Halogen wie Fluor, Chlor, Brom und Jod, bevorzugt Fluor, Chlor und Brom, besonders bevorzugt Chlor und Brom,
- Cyano,
- C₂- bis C₂₀-Alkylcarbonyloxy, bevorzugt C₂- bis C₈-Alkylcarbonyloxy wie Methylcarbonyloxy,
- C₃- bis C₂₀-Carboalkoxy, bevorzugt C₃- bis C₈-Carboalkoxy wie Carbomethoxy, Carboethoxy und Carbopropoxy,
- Formyl,
- C₂- bis C₂₀-Dialkylamino, bevorzugt Di-C₁-C₆-alkylamino, besonders Di-C₁-C₄-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino,
- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- Aryloxy wie Phenoxy, 1-Naphthoxy, 2-Naphthoxy, 1-Anthroxy, 2-Anthroxy und 9-Anthroxy, bevorzugt Phenoxy, 1-Naphthoxy und 2-Naphthoxy, besonders bevorzugt Phenoxy,
- Arylthio wie Phenylthio, 1-Naphthylthio, 2-Naphthylthio, 1-Anthrylthio, 2-Anthrylthio und 9-Anthrylthio, bevorzugt Phenylthio, 1-Naphthylthio und 2-Naphthylthio, besonders bevorzugt Phenylthio,
- Aroyl wie Phenoyl, 1-Naphthoyl, 2-Naphthoyl, 1-Anthroyl, 2-Anthroyl und 9-Anthroyl, bevorzugt Phenoyl, 1-Naphthoyl und 2-Naphthoyl, besonders bevorzugt Phenoyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- C₇- bis C₂₀-Aralkoxy, bevorzugt C₇- bis C₁₂-Phenylalkoxy wie Benzyloxy, 1-Phenethoxy, 2-Phenethoxy, 1-Phenyl-propoxy, 2-Phenyl-propoxy, 3-Phenyl-propoxy, 1-Phenyl-butoxy, 2-Phenyl-butoxy, 3-Phenyl-butoxy und 4-Phenyl-butoxy, besonders bevorzugt Benzyl, 1-Phenethoxy und 2-Phenethoxy,
- Arylsulfonyl wie Phenylsulfonyl, 1-Naphthylsulfonyl, 2-Naphthylsulfonyl, 1-Anthrylsulfonyl, 2-Anthrylsulfonyl und 9-Anthrylsulfonyl, bevorzugt Phenylsulfonyl, 1-Naphthylsulfonyl und 2-Naphthylsulfonyl, besonders bevorzugt Phenylsulfonyl,
- C₇- bis C₂₀-Aralkylthio bevorzugt C₇- bis C₁₂-Phenylalkylthio wie Benzylthio, 1-Phenethylthio, 2-Phenethylthio, 1-Phenyl-propylthio, 2-Phenyl-propylthio, 3-Phenyl-propylthio, 1-Phenyl-butylthio, 2-Phenyl-butylthio, 3-Phenyl-butylthio und 4-Phenyl-butylthio, besonders bevorzugt Benzylthio, 1-Phenethylthio und 2-Phenethylthio,
- und/oder ein gegebenenfalls ein- bis zweifach durch einen der folgenden Reste substituiertes Aryl;
- Chlorformyl
- Nitro
- sowie im Falle von den Verbindungen II noch zusätzlich ein oder zwei Hydroxyfunktionen
R¹
- -: C₅- bis C₂₀-Alkyl, bevorzugt C₅- bis C₁₈-Alkyl wie n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl, iso-Dodecyl und n-Tridecyl besonders bevorzugt C₅- bis C₁₂-Alkyl wie n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl,
R², R³
- Wasserstoff,
- C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl.

Als Beispiele für die Pyridinverbindungen der allgemeinen Formel III seien beispielsweise 3-n-Pentylpyridin, 3-n-Hexylpyridin, 3-n-Nonylpyridin, 2-Undecylpyridin und 2-Tridecylpyridin.

Beispiele für einzelne erfindungsgemäß einsetzbare Phenole II sind:
Phenol, o-Kresol, m-Kresol, p-Kresol, Xylenole, 4-Chlorphenol, Hydrochinon, Resorcin, 1-Naphthol, 2-Naphthol, 1,5-Dihydroxynaphthalin, 4,4'-Methylenbisphenol, 1,1-Bis-(4-hydroxyphenyl)-ethan, 2,2 -Bis-(4-hydroxyphenyl)-propan, 4,4'-Cyclohexyliden-bisphenol, 4,4'-Dihydroxydiphenylether, 4,4'-Dihydroxydiphenylsulfid, 4,4'-Dihydroxydiphenylsulfon, 4,4'-Dihydroxydiphenyl-N-methyl-amin, 4-Hydroxybenzophenon.

Als Chlorameisensäurearylester, die nach dem erfindungsgemäßen Verfahren hergestellt werden können, seien beispielsweise genannt:
Chlorameisensäurephenylester, -o-kresylester, -m-kresylester, -p-kresylester, -dimethylphenylester, -4-chlorphenylester, -naphthylester, Hydrochinon-, Resocrin-, 1,5-Dihydroxynaphthalin-bis-chloroformat, 4,4'-Methylenbisphenyl-, 1,1 -Bis-(4-hydroxyphenyl)-ethan-, 2,2-Bis-(4-hydroxyphenyl)-propan-, 4,4'-Cyclohexyliden-bisphenyl-, 4,4'-Dihydroxydiphenylether-bis-chloroformat, Benzophenon-4-chlorformiat.

Die Chlorameisensäurearylester, insbesondere der Chlorameisensäurephenylester, werden nach dem erfindungsgemäßen Verfahren in hoher Reinheit erhalten. Sie können daher für viele Zwecke als Rohprodukte weiterverwendet werden. Selbstverständlich können sie in bekannter Weise, beispielsweise durch Destillation oder Umkristallisation, auch weiter gereinigt werden.

Chlorameisensäurearylester sind wertvolle Zwischenprodukte, beispielsweise bei der Herstellung von Farbstoffen, wie Sirius-Farbstoffe (DE-A-23 25 088, Ullmanns Enzyklopädie der technischen Chemie, Band 4, Seite 105, 108 und 109, Urban und Schwarzenberg, 3. Auflage, Berlin-München., 1953), zur Herstellung von Polycarbonatkunststoffen, Pflanzenschutzmitteln und zur Herstellung von Bakteriziden. Diese Verwendungszwecke sind dem Fachmann bekannt und beispielsweise beschrieben in DE-A-21 31 555, DE-A-12 13 419 und Ullmanns Enzyklopädie der technischen Chemie, 4. Auflage, Band 9, Seite 383, Verlag Chemie 1975.

### Beispiele

### Beispiel 1

### Herstellung von Phenylchlorformiat

In eine Mischung von 188 g (2 Mol) Phenol und 9,3 g (0,04 Mol) 2-Undecylpyridin wurde bei einer Temperatur von 110°C unter Rühren innerhalb von 8 Std. 210 g (2,12 Mol) Phosgen eingeleitet. Zur Vervollständigung der Reaktion wurde noch 1 Std. bei 110°C nachgerührt, dann wurde das Produkt mittels Durchleiten von Stickstoff bei 60 bis 80°C innerhalb von 2 Std. von überschüssigem Phosgen befreit.

Durch Destillation des Rohproduktes wurden 279 g Phenylchlorformiat erhalten, entsprechend einer Ausbeute von 89 %.

Der flüssige Rückstand aus der Destillation wurde ein weiteres Mal mit 188 g (2 Mol) Phenol versetzt und die Umsetzung wie beschrieben durchgeführt.

Nach Destillation wurden 297 g Phenylchlorformiat erhalten, entsprechend einer Ausbeute von 95 %.

### Beispiel 2

### Herstellung von Phenylchlorformiat

In eine Mischung von 188 g (2 Mol) Phenol und 4,1 g (0,02 Mol) 3-Nonylpyridin wurden bei einer Temperatur von 100°C unter Rühren innerhalb von 5 Std. 206 g (2,08 Mol) Phosgen eingeleitet. Zur Vervollständigung der Reaktion wurde noch 1 Std. bei 100°C nachgerührt, dann wurde das Produkt mittels Durchleiten von Stickstoff bei 60 bis 80°C innerhalb von 2 Std. von überschüssigem Phosgen befreit.

Durch Destillation des Rohproduktes wurden 285 g Phenylchlorformiat erhalten, entsprechend einer Ausbeute von 91 %.

### Beispiel 3

### Herstellung von Phenylchlorformiat

In eine Mischung von 9,3 g (0,04 Mol) 2-Undecylpyridin und 20 g Phenylchlorformiat wurde bei einer Temperatur von 110°C unter Rühren innerhalb von 8 Std. gleichzeitig 188 g (2 Mol) Phenol und 213 g (2,15 Mol) Phosgen zugegeben. Zur Vervollständigung der Reaktion wurde noch 2 Std. bei 110°C nachgerührt, dann wurde das Produkt mittels Durchleiten von Stickstoff bei 60 bis 80°C innerhalb von 2 Std. von überschüssigem Phosgen befreit.

Durch Destillation des Rohproduktes wurden 285 g Phenylchlorformiat erhalten, entsprechend einer Ausbeute von 91 %.

Der flüssige Rückstand aus der Destillation wurde ein weiteres Mal mit Phenol und Phosgen wie beschrieben umgesetzt.

Nach Destillation wurden 294 g Phenylchlorformiat erhalten, entsprechend einer Ausbeute von 94 %.

### Beispiel 4 (Vergleichsbeispiel analog US-A-3 211,776; Beispiel 8)

### Herstellung von Phenylchlorformiat

In eine Mischung von 188 g (2 Mol) Phenol und 9,3 g (0,1 Mol) 3-Methylpyridin wurde bei einer Temperatur von 80°C unter Rühren innerhalb von 6 Std. 210 g (2,12 Mol) Phosgen eingeleitet. Zur Vervollständigung der Reaktion wurde noch 1 Std. bei 80°C nachgerührt, dann wurde das Produkt mittels Durchleiten von Stickstoff bei 60 bis 80°C innerhalb von 2 Std. von überschüssigem Phosgen befreit.

Durch Destillation des Rohproduktes wurden 244 g Phenylchlorformiat erhalten, entsprechend einer Ausbeute von 78 %.

Der Destillationsrückstand enthielt einen hohen Anteil an Feststoffen.

## Patentansprüche

1. Verfahren zur Herstellung von Chlorameisensäurearylestern der allgemeinen Formel I 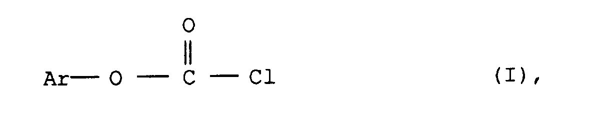 in der
Ar ein gegebenenfalls ein- bis fünffach durch C₁- bis C₂₀-Alkyl, C₂- bis C₂₀-Alkenyl, C₃- bis C₂₀-Cycloalkyl, C₄- bis C₂₀-Alkyl-cycloalkyl, C₁- bis C₂₀-Halogenalkyl, C₁- bis C₂₀-Alkoxy, C₁- bis C₂₀-Alkylthio, Halogen, Cyano, C₂- bis C₂₀-Alkylcarbonyloxy, C₃- bis C₂₀-Carboalkoxy, Formyl, C₂- bis C₂₀-Dialkylamino, Aryl, Aryloxy, Arylthio, Aroyl, C₇- bis C₂₀-Aralkyl, C₇- bis C₂₀-Aralkoxy, Arylsulfonyl und/oder C₇- bis C₂₀-Aralkylthio und/oder ein- bis zweifach durch Chlorformyl und/oder Nitro substituiertes Aryl
bedeutet, aus Phosgen und Phenolen der allgemeinen Formel II
Ar-OH (II),
in der Ar die obengenannten Bedeutungen hat sowie gegebenenfalls ein- bis zweifach Hydroxygruppen trägt, dadurch gekennzeichnet, daß man die Umsetzung bei einer Temperatur von 40 bis 250°C und Drücken von 0,01 bis 50 bar in Gegenwart von Pyridinverbindungen der allgemeinen Formel III 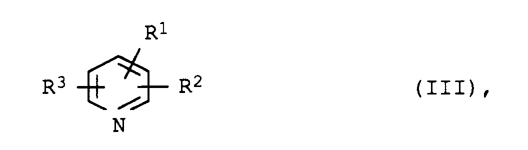 in der
R¹ C₅- bis C₂₀-Alkyl und
R², R³ Wasserstoff oder C₁- bis C₂₀-Alkyl
bedeuten, durchgeführt.

2. Verfahren nach Anspruch 1, dadurch gekennzeichnet, daß man Pyridinverbindung III verwendet, in der R¹ C₅- bis C₁₈-Alkyl und R² und R³ Wasserstoff bedeuten.

3. Verfahren nach Anspruch 1, dadurch gekennzeichnet, daß man die Pyridinverbindungen III in Mengen von 0,01 bis 25 Mol-% pro Äquivalent organische Hydroxylgruppen im Phenol verwendet.

4. Verfahren nach Anspruch 1, dadurch gekennzeichnet, daß man bei einer Temperatur zwischen 60 und 150°C arbeitet.
